# FASCICULE DE BREVET EUROPEEN

(11) **EP 1 149 829 B1**
(45) Date de publication et mention de la délivrance du brevet: **15.01.2003**
(21) Numéro de dépôt: 01400834.6
(22) Date de dépôt: 02.04.2001
(51) Int. Cl.: C07C 319/24

(54) **Procédé de fabrication d'oléfines sulfurées**
Verfahren zur Herstellung von sulfurierten Olefinen
Process for the preparation of sulfurized olefins

(30) Priorité: 28.04.2000 FR 0005499
(43) Date de publication de la demande: 31.10.2001
(73) Titulaire: Atofina, 92091 Paris La Défense (FR)
(72) Inventeur: Devaux, Jean-François, 64110 Jurancon (FR)
(74) Mandataire: Leboulenger, Jean

(56) Documents cités:
- EP-A- 0 342 454
- EP-A- 0 554 011
- EP-A- 0 656 414
- DATABASE CHEMABS [en ligne] CHEMICAL ABSTRACTS SERVICE, COLUMBUS, OHIO, US; STN, CAPLUS accession no. 1987:35910, XP002169301 & JP 61 183392 A (DAINIPPON INK AND CHEMICALS INC) 16 août 1986 (1986-08-16)

## Description

La présente invention concerne le domaine des oléfines sulfurées et a plus particulièrement pour objet un nouveau procédé de production d'oléfines sulfurées faiblement colorées par sulfuration au moyen de soufre et d'hydrogène sulfuré.

Les oléfines sulfurées sont des produits largement utilisés pour la sulfuration des catalyseurs et comme additifs pour lubrifiants ou pour élastomères. Ces produits sont composés essentiellement de mélanges de sulfures, disulfures et polysulfures organiques.

De nombreux procédés de production d'oléfines sulfurées et de polysulfures organiques sont connus de l'homme de l'art. Une première famille de procédés consiste à faire réagir un mercaptan et du soufre en présence d'un catalyseur basique. Ces procédés, décrits par exemple dans les brevets FR 2 607 496 et FR 2 630 104, sont coûteux car ils nécessitent l'utilisation de mercaptans qui doivent eux-mêmes être produits à partir d'oléfines ou d'alcools.

Le procédé décrit dans le brevet EP 342 454 pour produire des dialkyldisulfures et dialkylpolysulfures à partir d'oléfines est en fait un procédé en deux étapes où l'on effectue, dans un premier temps, une réaction H₂S + oléfine en présence d'un catalyseur solide pour former un mercaptan et où, dans un deuxième temps, ce mercaptan est mis en présence de soufre et d'un autre catalyseur hétérogène pour former un polysulfure. Ce procédé présente l'inconvénient de nécessiter deux étapes successives (2 réacteurs différents) avec des températures élevées.

D'autres procédés permettant d'obtenir des oléfines sulfurées ont été proposés :
**1)** La réaction oléfine + soufre en l'absence d'H₂S produit généralement des produits colorés. Pour éviter cet inconvénient, il a été proposé d'opérer en présence d'eau ou d'effectuer des lavages aqueux, mais ceci pose des problèmes de séparation de la phase aqueuse et d'élimination des effluents aqueux. De tels procédés sont décrits dans les brevets US 5 338 468, WO 92/03524, WO 92/00367, WO 97/24416, EP 714 970, EP 714 971 et FR 2 757 534. Dans tous ces procédés des températures élevées sont nécessaires pour mener à bien la réaction. Le brevet EP 201 197 décrit la réaction soufre + oléfine à une température de 140 à 180 °C.
**2)** La réaction oléfine + soufre + H₂S a été décrite. L'absence de catalyseur comme dans le brevet US 4 119 550 oblige à utiliser des températures et pressions très élevées. Les brevets EP 889 030, US 4 119 549, US 4 191 659, US 4 584 113 et JP 11-246518 décrivent cette réaction avec un catalyseur homogène, qui est difficile à éliminer en fin de réaction. Le brevet EP 554 011 décrit cette même réaction en présence notamment de catalyseurs hétérogènes qui ont une efficacité modérée en terme de conversion à 110°C.

Il a maintenant été trouvé un nouveau procédé de production d'oléfines sulfurées par sulfuration au moyen de soufre et d'H₂S qui fournit des produits limpides et faiblement colorés et permet de réaliser la réaction en une seule étape.

Le procédé selon l'invention est caractérisé en ce que la réaction est effectuée en une seule étape en présence d'une zéolite à pores de taille moyenne ou grande (0,5 à 0,8 nm).

La zéolite à utiliser selon l'invention est un aluminosilicate caractérisé par une grande surface spécifique et une taille de pore précise. La formule chimique générale est M_{2/n}O.Al₂O₃.y SiO₂.w H₂O où M est le cation, n sa valence, w la quantité d'eau intracristalline et y est supérieur ou égal à 2. La zéolite peut être échangée par des cations alcalins comme par exemple Na⁺, Li ⁺, K⁺ ou Cs⁺, ou alcalino-terreux comme par exemple Mg²⁺ ou Ca²⁺, ou des cations métalliques comme par exemple Ag⁺, Co²⁺, Ni²⁺, Mo^{2+ ou 3+}, Fe^{2+ ou 3+}, Cr³⁺, La³⁺. Elle peut aussi être échangée par des ions ammoniums ou par l'ion H⁺.

Bien que beaucoup de facteurs puissent influencer l'activité catalytique de ces zéolites, les trois plus importants sont : la structure du squelette et sa taille de pores, le ratio silice / alumine dans le squelette et la nature des cations. Les zéolites selon l'invention sont les zéolites à pores de taille moyenne ou grande, dans la gamme de 0,5 à 0,8 nm. De préférence, ce sont les zéolites de type X, Y, L ou mordénite, et plus préférentiellement de type Y. Ces zéolites sont décrites dans le Handbook of Molecular Sieves, R. Szostak, Van Nostrand and Reinhold, New York 1992. Par exemple, l'unité de base du type X a typiquement une composition chimique à l'état hydraté de Na₈₆.[(AlO₂)₈₆.(SiO₂)₁₀₆].264 H₂O avec des pores de diamètre 0,74 nm; l'unité de base du type Y a typiquement une composition chimique à l'état hydraté de Na₅₆.[(AlO₂)₅₆.(SiO₂)₁₃₆].264 H₂O avec des pores de diamètre 0,74 nm; l'unité de base du type L a typiquement une composition chimique à l'état hydraté K₉.[(AlO₂)₉.(SiO₂)₂₇].22 H₂O avec des pores de diamètre 0,71 nm, et l'unité de base de la mordénite a typiquement une composition chimique à l'état hydraté de Na₈.[(AlO₂)₈.(SiO₂)₄₀].24 H₂O avec des pores de plus grand diamètre 0,7 nm.

Le cation initial provenant de la synthèse peut être échangé totalement ou partiellement par des cations alcalins, alcalino-terreux, métalliques, ammonium ou des protons.

Les oléfines à utiliser dans le procédé selon l'invention peuvent être choisies dans une très large gamme. Elles contiennent au moins une double liaison carbone-carbone qui n'est pas aromatique. Elles peuvent généralement être représentées par la formule : 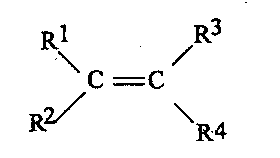 dans laquelle les symboles R¹, R², R³, R⁴, identiques ou différents, représentent chacun un atome d'hydrogène, un radical aryle ou un radical alkyle, linéaire, ramifié ou cyclique, contenant de 1 à 20 atomes de carbones et pouvant comporter une ou plusieurs insaturations et/ou groupes aromatiques et/ou groupes OR⁵, SR⁵, NR⁵R⁶, CN, COR⁵, COOR⁵, CONR⁵R⁶, SiR⁵R⁶R⁷, Si(OR⁵)R⁶R⁷, Si(OR⁵)(OR⁶)R⁷, Si(OR⁵)(OR⁶)OR⁷, SO₂R⁵, SO₃R⁵, POR⁵R⁶, OP(O)(OR⁵)(OR⁶), NO₂ ou halogène, chacun des symboles R⁵, R⁶ et R⁷ désignant indépendamment un atome d'hydrogène ou un radical alkyle, cycloalkyle ou aryle comportant éventuellement une ou plusieurs insaturations.

Deux des symboles R¹, R², R³, R⁴, peuvent aussi représenter un groupe alkylène éventuellement substitué, c'est-à-dire que la double liaison carbone-carbone peut être incluse dans un cycle, comme par exemple dans le cyclohexène, le cyclopentadiène, le dicyclopentadiène. L'oléfine de l'invention peut également être un acide gras insaturé ou polyinsaturé, un ester d'acide gras insaturé ou polyinsaturé, un dérivé d'acide gras contenant au moins une double liaison, ou un mélange de ces derniers. Par exemple, ce peut être l'acide oléique, linoléique, linolénique, palmitoléique ou leurs esters d'origine naturelle comme les triglycérides ou d'origine synthétique comme, par exemple, les esters d'alcools aliphatiques ou de polyols. Ces acides et esters gras peuvent être utilisés isolément ou en mélange comme dans les corps gras naturels, les huiles ou graisses animales ou végétales ou leurs produits dérivés. On peut citer par exemple l'huile de tournesol, de soja, de colza, de son de riz, de ricin, de suif, de tall oil. Dans le cadre de l'invention, les corps gras naturels ou leurs dérivés peuvent contenir une certaine proportion d'acides ou d'esters saturés, qui se comportent comme des solvants dans les conditions de l'invention. L'oléfine selon l'invention peut être également un terpène, comme par exemple le pinène, le menthène, le limonène.

Des mélanges de plusieurs oléfines peuvent aussi être utilisés. A titre d'exemple, on peut mentionner le mélange d'un corps gras naturel avec une oléfine aliphatique non fonctionnalisée.

De préférence, on utilisera une oléfine telle que l'isobutylène, le diisobutylène, le triisobutylène, le tripropylène, le tétrapropylène, un acide gras, un ester gras, un mélange d'acides ou d'esters gras, ou une huile animale ou végétale éventuellement en mélange avec une oléfine aliphatique non fonctionnalisée.

De plus, les oléfines utilisées selon l'invention peuvent être diluées dans des solvants. En fin de réaction, ces solvants partent dans les évents ou sont séparés par distillation. De tels solvants peuvent être par exemple des hydrocarbures aliphatiques saturés, comme le méthane, l'éthane, le propane, un butane ou un pentane. Le fait d'utiliser de tels mélanges d'oléfines et d'hydrocarbures saturés peut substantiellement améliorer l'économie du procédé selon l'invention dans la mesure où de telles charges peuvent être moins coûteuses qu'une charge d'oléfine relativement pure. Par exemple, on pourra utiliser une coupe contenant des hydrocarbures saturés et insaturés de 4 atomes de carbone à la place d'isobutylène pur.

Le soufre peut être utilisé sous forme solide, en pastille, en poudre ou sous forme liquide. Le ratio molaire soufre / oléfine peut aller de 0,4 : 1 à 2,5 : 1 et est de préférence compris entre 0,5 : 1 et 2 : 1.

Le ratio molaire H₂S / oléfine peut varier dans une large gamme (de 0,5 : 1 à 5 : 1, voire plus), mais de préférence on utilise le minimum d'H₂S nécessaire pour que la réaction s'opère de façon satisfaisante, soit un ratio H₂S/oléfine compris entre 0,5 : 1 et 2 : 1.

Le procédé selon l'invention peut être mis en oeuvre en discontinu (batch) ou en continu.

L'efficacité catalytique de la zéolite se manifeste généralement à partir d'une quantité minimale de 0,5 % en poids par rapport à la quantité d'oléfine. Dans la plupart des cas, la quantité maximale utile est de l'ordre de 50 % en poids. Dans un procédé discontinu (batch), la quantité préférée est comprise entre 5 et 30 %.

Dans le cas d'une mise en oeuvre du procédé en continu, une charge de catalyseur peut être utilisée pendant de longues périodes pour produire de grandes quantités de produits et le ratio massique de catalyseur / oléfine n'a plus grande signification.

Le procédé selon l'invention peut être mis en oeuvre dans n'importe quel équipement adapté, par exemple, dans un réacteur, muni d'un agitateur, où le catalyseur est en suspension dans le milieu réactionnel liquide. Il peut aussi être mis en oeuvre au moyen d'un réacteur tubulaire dans lequel le catalyseur est disposé en lit fixe, en lit mobile ou en lit expansé. De préférence on utilisera un réacteur en lit fixe.

La réaction proprement dite peut avoir lieu dans un large domaine de température suivant les oléfines utilisées et le catalyseur employé. Elle est en général effectuée à une température comprise entre 20 et 180°C, de préférence entre 70 et 150°C.

La réaction est conduite à une pression adaptée à la conversion de l'oléfine. On opère avantageusement entre 1 et 50 bars absolus, de préférence entre 1 et 20 bars absolus. La pression peut varier au cours de la réaction notamment en fonction du profil de température et de l'avancement de la réaction.

Avantageusement, après une phase sous pression en réacteur fermé où l'oléfine est convertie, le ciel du réacteur est mis à pression atmosphérique ou en dépression de façon à éliminer l'H₂S excédentaire et à finir de convertir le mercaptan formé. On peut envisager dans cette dernière phase d'introduire un gaz inerte (par exemple du méthane, de l'air ou de l'azote) de façon à entraîner les composés volatiles résiduels tel l'H₂S ou l'oléfine résiduelle.

Dans le cas d'une réaction par batch, en fin de réaction, le catalyseur peut être récupéré par simple filtration et réutilisé.

Si l'on désire diminuer son odeur, le stabiliser ou réduire sa corrosivité, le produit soufré peut être traité par toute méthode connue par l'homme de l'art. De telles méthodes sont décrites par exemple dans les brevets JP 58140063, US 5 155 275, US 5 206 439, US 5 218 147, US 5 403 961, US 5 457 234, US 5 530 163, US 5 559 271, US 5 091 112, US 5 174 922, US 5 208 382, US 5 242 613, EP 76376, et EP 933 358.

Les exemples suivants illustrent l'invention sans la limiter.

### EXEMPLE 1

Dans un réacteur en acier inoxydable de 1 litre inerté à l'azote, on a introduit 60 g de zéolite LZY-52 sous forme d'extrudés (vendue par UOP), qui est une zéolite de type Y échangée contenant typiquement 10,4% d'oxyde de sodium, 0,3% d'oxyde de calcium, 0,2% d'oxyde de fer III, 66,5% de silice et 20,8% d'alumine et présentant une porosité de 0,74 nm et une surface spécifique d'environ 820 m²/g. On a introduit ensuite 153 g de soufre (4,8 mol) et 336 g de diisobutylène (3,0 mol). On a alors ajouté 82 g d'H₂S (2,4 mol) en 40 minutes à 20°C. On a porté la température à 88°C et laissé réagir pendant 7 heures puis 4 heures supplémentaires. La pression maximale atteinte a été de 17,5 bars absolus et était de 10,5 bars en fin de réaction.

L'analyse de prélèvements montre que le mélange réactionnel contenait après 7 heures 9,2 % de diisobutylène et 6,5 % en masse de mercaptan (exprimé par rapport à une masse moléculaire de 146), puis après les 4 heures supplémentaires 3,6 % de diisobutylène et 6,2 % en masse de mercaptan.

Après filtration, on a obtenu 474 g d'une huile jaune limpide dont la teneur en soufre total a été dosée à 37% massique. Aucun dépôt de soufre solide n'a été observé ni dans la phase liquide, ni sur le filtre.

### EXEMPLE 2 (comparatif)

Les conditions de l'exemple 1 ont été reproduites en utilisant comme catalyseur une alumine activée acide de type 504C (150 µm) vendue par Aldrich. On a laissé réagir 11 heures à 90°C. La pression maximale atteinte a été de 25 bars absolus et était de 23 bars en fin de réaction.

L'analyse du mélange réactionnel obtenu montrait une teneur en diisobutylène non converti de 34% et une teneur en mercaptan de 5,6%.

Après filtration, on a obtenu 355 g d'une huile jaune et observé sur le filtre un important dépôt de soufre solide.

### EXEMPLE 3

Dans le même réacteur qu'à l'exemple 1, on a introduit 30 g de zéolite LZY-54 sous forme d'extrudés (vendue par UOP), qui est une zéolite de type Y échangée contenant typiquement 10 % d'oxyde de sodium, 0,23 % d'oxyde de fer III, 66 % de silice et 21 % d'alumine et présentant une porosité de 0,74 nm et une surface spécifique d'environ 750 m²/g. On a introduit ensuite 28,4 g de soufre (0,89 mol) et 250 g d'oléate de méthyle technique vendu par FINA CHEMICALS et contenant 55 % massique d'oléate de méthyle (soit 0,46 mol). On a alors porté la température à 130°C et ajouté en continu de l'hydrogène sulfuré pour maintenir une pression de 13 bars absolus. Après 9 heures de réaction dans ces conditions, la consommation d'hydrogène sulfuré était de 20 g.

Après 1 heure et demie de stripping à l'azote à pression atmosphérique, la teneur en oléate de méthyle était de 4,6 %. Après filtration, on a obtenu 256 g d'une huile jaune-orangé. Aucun dépôt de soufre solide n'a été observé ni dans la phase liquide, ni sur le filtre.

## Revendications

1. Procédé de fabrication d'oléfines sulfurées à partir d'oléfine(s), de soufre et d'hydrogène sulfuré, **caractérisé en ce que** la réaction est effectuée en une seule étape en présence d'un catalyseur solide à base de zéolite à pores de taille moyenne ou grande, dans la gamme de 0,5 à 0,8 nm.

2. Procédé selon la revendication 1 dans lequel le catalyseur solide est une zéolite de type X, Y, L ou mordénite.

3. Procédé selon la revendication 1 à 2 dans lequel le catalyseur solide est une zéolite de type Y.

4. Procédé selon l'une des revendications 1 à 3 dans lequel le rapport molaire soufre / oléfine(s) va de 0,4:1 à 2,5:1.

5. Procédé selon l'une des revendications 1 à 4 dans lequel le rapport molaire H₂S / oléfine(s) va de 0,5:1 à 5:1.

6. Procédé selon l'une des revendications 1 à 5 dans lequel le catalyseur est utilisé en une quantité allant de 0,5 à 50 % en poids par au poids d'oléfine(s).

7. Procédé selon l'une des revendications 1 à 6 dans lequel on opère à une température allant de 20 à 180°C.

8. Procédé selon l'une des revendications 1 à 7 dans lequel on opère à une pression allant de 1 à 50 bars absolus.

9. Procédé selon l'une des revendications 1 à 8 dans lequel la ou les oléfines sont choisies parmi celles de formule : 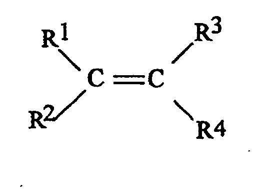 dans laquelle les symboles R¹, R², R³, R⁴, identiques ou différents, représentent chacun un atome d'hydrogène, un radical aryle ou un radical alkyle, linéaire, ramifié ou cyclique, contenant de 1 à 20 atomes de carbones et pouvant comporter une ou plusieurs insaturations et/ou groupes aromatiques et/ou groupes OR⁵, SR⁵, NR⁵R⁶, CN, COR⁵, COOR⁵, CONR⁵R⁶, SiR⁵R⁶R⁷, Si(OR⁵)R⁶R⁷, Si(OR⁵)(OR⁶)R⁷, Si(OR⁵)(OR⁶)OR⁷, SO₂R⁵, SO₃R⁵, POR⁵R⁶, OP(O)(OR⁵)(OR⁶), NO₂ ou halogène, chacun des symboles R⁵, R⁶ et R⁷ désignant indépendamment un atome d'hydrogène ou un radical alkyle, cycloalkyle ou aryle comportant éventuellement une ou plusieurs insaturations, deux des symboles R¹, R², R³, R⁴, pouvant aussi représenter un groupe alkylène éventuellement substitué.

10. Procédé selon la revendication 9 dans lequel on utilise l'isobutylène, le diisobutylène, le triisobutylène, le tripropylène ou le tétrapropylène.

11. Procédé selon la revendication 9 dans lequel on utilise un acide gras, un ester gras, un mélange d'acides ou d'esters gras, ou une huile animale ou végétale.

12. Procédé selon la revendication 9 dans lequel on utilise un mélange d'oléfine aliphatique non fonctionnalisée avec un acide gras, un ester gras ou une huile animale ou végétale.

## Claims

1. Process for manufacturing sulphurized olefins from olefin(s), sulphur and hydrogen sulphide, **characterized in that** the reaction is carried out in a single step in the presence of a solid catalyst based on zeolite with a medium or large pore size, in the range from 0.5 to 0.8 nm.

2. Process according to Claim 1, in which the solid catalyst is a zeolite of type X, Y or L, or mordenite.

3. Process according to Claim 1 or 2, in which the solid catalyst is a zeolite of type Y.

4. Process according to one of Claims 1 to 3, in which the sulphur/olefin(s) molar ratio is from 0.4:1 to 2.5:1.

5. Process according to one of Claims 1 to 4, in which the H₂S/olefin(s) molar ratio is from 0.5:1 to 5:1.

6. Process according to one of Claims 1 to 5, in which the amount of catalyst used is from 0.5 to 50% by weight, with respect to the weight of olefin(s).

7. Process according to one of Claims 1 to 6, which is worked at a temperature ranging from 20 to 180°C.

8. Process according to one of Claims 1 to 7, which is worked at a pressure ranging from 1 to 50 bar absolute.

9. Process according to one of Claims 1 to 8, in which the olefin(s) are chosen from those of the formula: 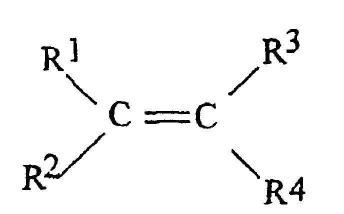 in which each of the symbols for R¹, R², R³ and R⁴, which are identical or different, represents a hydrogen atom, an aryl radical or an alkyl radical, linear, branched or cyclic, which contains from 1 to 20 carbon atoms and may contain one or more unsaturations and/or aromatic groups and/or OR⁵, SR⁵, NR⁵R⁶, CN, COR⁵, COOR⁵, CONR⁵R⁶, SiR⁵R⁶R⁷, Si(OR⁵)R⁶R⁷, Si(OR⁵)(OR⁶)R⁷, Si(OR⁵)(OR⁶)OR⁷, SO₂R⁵, SO₃R⁵, POR⁵R⁶, OP(O)(OR⁵)(OR⁶), NO₂ or halogen groups, each of the symbols for R⁵, R⁶ and R⁷ denoting independently a hydrogen atom or an alkyl, cycloalkyl or aryl radical optionally containing one or more unsaturations, and two of the symbols for R¹, R², R³ and R⁴ may also represent an unsubstituted or substituted alkylene group.

10. Process according to Claim 9, in which use is made of isobutylene, diisobutylene, triisobutylene, tripropylene or tetrapropylene.

11. Process according to Claim 9, in which use is made of a fatty acid, a fatty ester, a mixture of fatty esters or acids, or an oil of animal or vegetable origin.

12. Process according to Claim 9, in which use is made of a mixture of non-functionalized aliphatic olefin with a fatty acid, a fatty ester or an oil of animal or vegetable origin.

## Patentansprüche

1. Verfahren zur Herstellung sulfurierter Olefine ausgehend von Olefin(en), Schwefel und Schwefelwasserstoff, **dadurch gekennzeichnet, daß** die Reaktion in einem einzigen Schritt in Gegenwart eines festen Katalysators auf Basis von Zeolith mit Poren mittlerer oder großer Größe im Bereich von 0,5 bis 0,8 nm durchgeführt wird.

2. Verfahren nach Anspruch 1, wobei der feste Katalysator ein Zeolith vom Typ X, Y, L oder Mordenit ist.

3. Verfahren nach Anspruch 1 oder 2, wobei der feste Katalysator ein Zeolith vom Typ Y ist.

4. Verfahren nach einem der Ansprüche 1 bis 3, wobei das Molverhältnis Schwefel/Olefin(e) von 0,4:1 bis 2,5:1 reicht.

5. Verfahren nach einem der Ansprüche 1 bis 4, wobei das Molverhältnis H₂S/Olefin(e) von 0,5:1 bis 5:1 reicht.

6. Verfahren nach einem der Ansprüche 1 bis 5, wobei der Katalysator in einer Menge von 0,5 bis 50 Gew.-%, bezogen auf das Gewicht des oder der Olefine, verwendet wird.

7. Verfahren nach einem der Ansprüche 1 bis 6, wobei man bei einer Temperatur von 20 bis 180 °C verfährt.

8. Verfahren nach einem der Ansprüche 1 bis 7, wobei man bei einem Druck von 1 bis 50 bar absolut verfährt.

9. Verfahren nach einem der Ansprüche 1 bis 8, wobei das oder die Olefine aus Olefinen der Formel 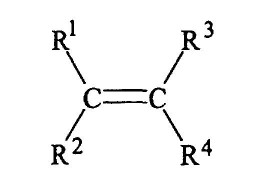 ausgewählt werden, wobei in der Formel die Symbole R¹, R², R³ und R⁴, identisch oder verschieden, jeweils ein Wasserstoffatom, einen Arylrest, einen linearen, verzweigten oder ringförmigen Alkylrest mit 1 bis 20 Kohlenstoffatomen darstellen und ein oder mehrere ungesättigte Bindungen und/oder aromatische Gruppen und/oder Gruppen vom Typ OR⁵, SR⁵, NR⁵R⁶, CN, COR⁵, COOR⁵, CONR⁵R⁶, SiR⁵R⁶R⁷, Si(OR⁵)R⁶R⁷, Si(OR⁵)(OR⁶)R⁷, Si(OR⁵)(OR⁶)OR⁷, SO₂R⁵, SO₃R⁵, POR⁵R⁶, OP(O)(OR⁵)(OR⁶) und NO₂ oder ein Halogen umfassen können, wobei jedes der Symbole R⁵, R⁶ und R⁷, unabhängig voneinander, ein Wasserstoffatom oder einen Alkyl-, Cycloalkyl- oder Arylrest, der gegebenenfalls ein oder mehrere ungesättigte Bindungen enthält, bezeichnet, wobei zwei der Symbole R¹, R², R³ und R⁴ auch eine gegebenenfalls substituierte Alkylengruppe darstellen können.

10. Verfahren nach Anspruch 9, wobei man Isobutylen, Diisobutylen, Triisobutylen, Tripropylen oder Tetrapropylen einsetzt.

11. Verfahren nach Anspruch 9, wobei man eine Fettsäure, einen Fettsäureester, eine Mischung von Fettsäuren oder Fettsäureestern oder ein tierisches oder pflanzliches Öl einsetzt.

12. Verfahren nach Anspruch 9, wobei man eine Mischung eines nichtfunktionalisierten aliphatischen Olefins mit einer Fettsäure, einem Fettsäureester oder einem tierischen oder pflanzlichen Öl einsetzt.
